# EUROPÄISCHE PATENTSCHRIFT

(11) **EP 1 307 262 B1**
(45) Veröffentlichungstag und Bekanntmachung des Hinweises auf die Patenterteilung: **06.10.2004**
(21) Anmeldenummer: 01955345.2
(22) Anmeldetag: 12.07.2001
(51) Int. Cl.: A61P 1/00, G01N 33/50

(54) **VERFAHREN ZUM AUFFINDEN VON VERBINDUNGEN, WELCHE ZUR BEHANDLUNG UND/ODER PROPHYLAXE VON OBESITAS GEEIGNET SIND**
METHOD FOR LOCATING COMPOUNDS WHICH ARE SUITABLE FOR THE TREATMENT AND/OR PROPHYLAXIS OF OBESITY
PROCEDE POUR TROUVER DES COMPOSES APTES AU TRAITEMENT ET/OU A LA PROPHYLAXIE DE L'OBESITE

(30) Priorität: 20.07.2000 DE 10035227
(43) Veröffentlichungstag der Anmeldung: 07.05.2003
(73) Patentinhaber: Solvay Pharmaceuticals GmbH, 30173 Hannover (DE)
(72) Erfinder: HEBEBRAND, Johannes, 35039 Marburg/Lahn (DE); ANTEL, Jochen, 31848 Bad Münder (DE); PREUSCHOFF, Ulf, 30916 Isernhagen N.B. (DE); DAVID, Samuel, 30559 Hannover (DE); SANN, Holger, 30629 Hannover (DE); WESKE, Michael, 31305 Burgdorf (DE)
(74) Vertreter: Bauriegel, Lutz
(86) Internationale Anmeldenummer: PCT/EP2001/008051
(87) Internationale Veröffentlichungsnummer: WO 2002/007821

(56) Entgegenhaltungen:
- WO-A-94/09813
- C. T. SUPURAN ET AL.: "Carbonic anhydrase inhibitors and their therapeutic potential" EXPERT OPINION ON THERAPEUTIC PATENTS, Bd. 10, Nr. 5, Seiten 575-600, XP001026091 in der Anmeldung erwähnt
- M. K. HELLERSTEIN: "De novo lipogenesis in humans: metabolic and regulatory aspects" EUROPEAN JOURNAL OF CLINICAL NUTRITION, Bd. 53, Nr. 1, Januar 1999 (1999-01), Seiten 53-65, XP001037679 in der Anmeldung erwähnt

## Beschreibung

Die vorliegende Erfindung betrifft ein Verfahren zum Auffinden von Verbindungen, welche zur Behandlung und/oder zur Prophylaxe von Obesitas geeignet sind. Weiterhin betrifft die Erfindung die Verwendung von Verbindungen, welche die Fähigkeit besitzen, die de-novo-Lipogenese in Säugetieren zu hemmen und welche im wesentlichen frei sind von auf das zentrale Nervensystem (= ZNS) gerichteten Wirkungen, zur Herstellung von Arzneimitteln für die Behandlung und/oder Prophylaxe von Obesitas.

Obesitas (= Fettleibigkeit) ist heute insbesondere in den entwickelten Industrienationen ein zunehmend ernsteres Problem für die Gesundheit der Bevölkerung, welches größtenteils durch unausgewogene und zu fettreiche Ernährung verursacht wird. Mit der Zunahme des prozentualen Bevölkerungsanteils übergewichtiger Menschen nehmen auch die Folgeerscheinungen der Obesitas zu, die von persönlicher Unzufriedenheit bis hin zu Herz-Kreislauf-Erkrankungen oder auch bestimmten Formen von Diabetes reichen. Es gibt daher bereits einige Therapieansätze, welche auf eine Behandlung oder Prophylaxe der Obesitas abzielen. Als Beispiel seien Lipase-hemmende Verbindungen genannt, welche die Fettspaltung im Darmtrakt reduzieren und auf diese Weise die Energieausbeute aus der aufgenommenen Nahrung herabsetzen. Nahrungsfette werden bei diesem Therapieansatz also wenigstens teilweise wieder unzersetzt ausgeschieden. Weitere neue Therapieansätze zur Behandlung und/oder Prophylaxe von Obesitas, welche die vorbekannten Therapieformen ergänzen können, sind jedoch wünschenswert.

Es wurde nun überraschend gefunden, daß sich Verbindungen, welche die de-novo-Lipogenese in Säugetieren, insbesondere Menschen, hemmen können, in vorteilhafter Weise zur wirkungsvollen Behandlung und/oder Prophylaxe von Obesitas eignen. Besonders gute Ergebnisse erzielt man dabei, wenn die vorgenannten Verbindungen über längere Zeiträume, beispielsweise über Zeiträume von mehreren Wochen, verabreicht werden.

Gegenstand der Erfindung ist ein Verfahren zum Auffinden von Verbindungen, welche zur Behandlung und/oder Prophylaxe von Obesitas geeignet sind, worin man solche Verbindungen auswählt, welche die Fähigkeit besitzen, die de-novo-Lipogenese in Säugetieren, insbesondere Menschen, zu hemmen. Ferner ist Gegenstand der Erfindung die Verwendung von Verbindungen, welche die Fähigkeit besitzen, die de-novo-Lipogenese in Säugetieren zu hemmen und welche insbesondere im wesentlichen frei sind von auf das ZNS gerichteten Wirkungen wie antikonvulsiven Wirkungen, zur Herstellung von Arzneimitteln für die Behandlung und/oder Prophylaxe von Obesitas.

Unter de-novo-Lipogenese (im folgenden mit DNL abgekürzt) wird der Aufbau körpereigener Fettsäuren aus Kohlenhydraten im Organismus von Säugetieren verstanden. Diese Aufbaureaktion findet im Cytosol von Körperzellen statt, basierend auf dem sogenannten Citronensäurezyklus oder Krebs-Martius-Zyklus. In einer körpereigenen biochemischen Reaktion wird in diesem Zyklus aus den beiden Bausteinen Pyruvat, welches aus Kohlenhydraten stammt, und Hydrogencarbonat über verschiedene Zwischenstufen - darunter Maleat, Fumarat und α-Ketoglutarat - letztlich Citrat aufgebaut. Sofern Citrat im Überschuß aufgebaut wird, kann dieses über die Zwischenstufe Acetyl-Coenzym A in freie Fettsäuren (= lipidische Folgeprodukte) verwandelt werden, welche Fette bilden und dann in Fettzellen (= Adipozyten) eingelagert werden können. Eine übermäßige Einlagerung von aus Fettsäuren gebildeten Fetten in Körperzellen kann ganz allgemein zur Obesitas führen. Am Citronensäurezyklus sind verschiedene Enzyme beteiligt. Der Stoffumsatz des Citratzyklusses hängt wesentlich von der Menge des zur Verfügung stehenden Hydrogencarbonates ab. Die Menge des zur Verfügung stehenden Hydrogencarbonates ist dabei wiederum abhängig von der Geschwindigkeit, mit der es aus Kohlendioxid gebildet werden kann. Diese Hydrogencarbonat liefernde Gleichgewichtsreaktion wird durch sogenannte Carboanhydrasen katalysiert. Von den bisher bekannten Carboanhydrasen und deren Isozymen sind in Säugetieren überwiegend die Carboanhydrase-Isozyme der Subtypen II (= CA II) sowie V (= CA V) an der Katalyse von Reaktionen beteiligt, welche Hydrogencarbonat für den Citronensäurezyklus zur Verfügung stellen. Insbesondere spielen Carboanhydrasen des Subtyps V eine Rolle, da diese in den Mitochondrien vorliegen. Auch der Citronensäurezyklus findet in den Mitochondrien statt.

Es sind verschiedene Möglichkeiten denkbar, die DNL in Säugetierzellen zu hemmen, welche alle darauf abzielen, den im Citronensäurezyklus anfallenden Stoffumsatz herabzusetzen. Dadurch kann die Konzentration an überschüssig produziertem Citrat, welches für den Aufbau von Fettsäuren zur Verfügung steht, vermindert werden. Entsprechend der vorliegenden Erfindung kann die DNL vorzugsweise gehemmt werden, indem die Carboanhydrasen gehemmt werden, welche die Hydrogencarbonat für den Citronensäurezyklus liefernden Reaktionen katalysieren. Vorzugsweise können für den erfindungsgemäßen Zweck die Carboanhydrasen der Subtypen II und/oder V, vorzugsweise CA V, gehemmt werden.

Verbindungen, welche unspezifisch Carboanhydrasen hemmen können (= unspezifische oder klassische CA-Inhibitoren) sind an sich bekannt und werden seit längerem auf verschiedenen therapeutischen Gebieten, hauptsächlich als Diuretika oder in der Ophthalmologie, eingesetzt. Ein Überblick über Einsatzgebiete solcher klassischen CA-Inhibitoren findet sich z. B. bei C. T. Supuran, Expert Opinion on Therapeutic Patents, 10(5)(2000) 575 - 600. Unter "spezifischen CA-Inhibitoren" sollen dagegen Verbindungen verstanden werden, die weitgehend nur einen CA-Subtyp (z. B. CA V) oder eine definierte Gruppe von CA-Subtypen hemmen. Die Verwendung von klassischen CA-Inhibitoren zur gezielten Behandlung und/oder Prophylaxe von Obesitas ist bislang nicht bekannt.

Allgemein wird angenommen, daß die überwiegende Mehrzahl bekannter Fälle von Obesitas auf den verhältnismäßig überhöhten Anteil körperfremder Fette an der aufgenommenen Nahrung zurückzuführen ist. In hochentwickelten Ländern wie den USA nehmen fettleibige Menschen bis zu 30 % ihrer Nahrung in Form von Fetten zu sich. Nach heutigen Erkenntnissen stammen zur Obesitas führende Fett-Einlagerungen demnach überwiegend aus übermäßig aufgenommenen Nahrungsfetten, welche durch eine Hemmung der auf den Kohlenhydrat-Metabolismus abzielenden DNL nicht beeinflußbar sind.

Obwohl also auch durch eine Hemmung der DNL eine verminderte Einlagerung von Fettsäuren in Adipozyten erreicht wird, wurde eine Hemmung der DNL wegen ihres an sich geringen Beitrages zur Einlagerung von Fettsäuren in Körperzellen bislang als ungeeigneter Ansatzpunkt zur Behandlung und/oder Prophylaxe von Obesitas beim Menschen angesehen. Eine Zusammenfassung der in dieser Richtung in der Fachwelt geäußerten Meinungen findet sich beispielsweise bei M. K. Hellerstein, European Journal of Clinical Nutrition 53 (1)(1999) 53 - 65. Diese in der Fachwelt vorherrschende Ansicht stand auch bislang der gezielten Suche nach Arzneimitteln zur Behandlung und/oder Prophylaxe von Obesitas, welche auf dem Prinzip der DNL-Hemmung beruhen, oder deren Entwicklung, im Wege.

Im Rahmen der vorliegenden Erfindung wurde nun überraschend gefunden, daß Verbindungen, welche die Fähigkeit besitzen, die DNL in Säugetieren, insbesondere Menschen, zu hemmen, wirkungsvoll zur Behandlung und/oder Prophylaxe von Obesitas eingesetzt werden können, insbesondere wenn diese Verbindungen über längere Zeiträume von beispielsweise über sechs Wochen an die betretrenden Patienten verabreicht werden. Demnach können über längere Zeiträume signifikante Reduzierungen des Körpergewichtes fettleibiger Personen durch die Hemmung der DNL erreicht werden, obwohl die DNL an sich nur einen relativ geringen Beitrag zu dem in Adipozyten eingelagerten Körperfett leistet. Durch eine Hemmung der DNL über längere Zeiträume kann sich dieser an sich geringe Effekt also derart akkumulieren, daß er insgesamt einen signifikanten Beitrag zur Reduzierung des Körpergewichtes leistet.

Am Beispiel der Verbindung Topiramat läßt sich zeigen, daß das erfindungsgemäße Verfahren tatsächlich dazu geeignet ist, zur Behandlung und/oder Prophylaxe von Obesitas geeignete Verbindungen gezielt aufzufinden. Topiramat ist ein aus der EP 0 138 441 A2 bekanntes Antiepileptikum. Von Topiramat ist weiterhin bekannt, daß es ein multifaktorielles pharmakologisches Wirkungsspektrum aufweist. So kann Topiramat die spannungsabhängigen Natriumkanäle blockieren und somit das Membranpotential von Zellen stabilisieren, es aktiviert die GABA-Rezeptoren und verstärkt damit die GABA-vermittelte Inhibition, es wirkt als Carboanhydrase-Inhibitor und schließlich vermag es die Kainat/AMPA-Rezeptoren, einen Subtyp der Glutamat-Rezeptoren, zu hemmen, wodurch das Auftreten von AMPA-induzierten Strömen gehemmt wird. Topiramat weist demnach ausgeprägte Wirkungen auf das ZNS auf. Es ist nicht bekannt, ob die antiepileptischen Eigenschaften von Topiramat auf die vorgenannten pharmakologisch relevanten Faktoren zurückzuführen sind.

Aus der WO 98/00130 ist bekannt, daß Topiramat auch pharmakologische Eigenschaften besitzt, welche es zur Behandlung von Obesitas geeignet erscheinen lassen. Diese Eigenschaften waren als Begleitwirkungen in Langzeitstudien an Epilepsiepatienten zufällig entdeckt worden. Es ist bislang nicht bekannt, auf welche pharmakologischen Eigenschaften von Topiramat der an Epilepsiepatienten beobachtete Gewichtsverlust zurückzuführen ist. Topiramat fällt unter den Umfang einer in der WO 98/00130 als zur Behandlung von Obesitas geeignet angegebenen Gruppe antikonvulsiv wirksamer Verbindungen einer allgemeinen Formel I.

Nach dem erfindungsgemäßen Verfahren konnte nun gezeigt werden, daß Topiramat ein potenter CA-Inhibitor ist, insbesondere ein potenter Inhibitor von in Säugetieren vorkommenden Carboanhydrasen der Subtypen II und V, und daß Topiramat die DNL in Säugetierzellen wirkungsvoll zu hemmen vermag. Somit konnte mit Hilfe des erfindungsgemäßen Verfahrens erstmals belegt werden, daß die bislang unerklärlichen, zum Gewichtsverlust in Epilepsiepatienten führenden pharmakologischen Begleitwirkungen von Topiramat im wesentlichen auf dessen Fähigkeit beruhen, die DNL in Säugetieren wirkungsvoll zu hemmen. Es ist demnach zu erwarten, daß mit Hilfe des erfindungsgemäßen Verfahrens zukünftig Verbindungen aufgefunden werden können, welche zur Behandlung und/oder Prophylaxe von Obesitas geeignet sind. Mit dem erfindungsgemäßen Verfahren eröffnet sich nun erstmals die Möglichkeit, nach dem Prinzip der DNL-Hemmung wirkende Verbindungen mit pharmakologischer Potenz verhältnismäßig rasch und einfach aufzufinden. Dank der vorliegenden Erfindung können nach dem vorgenannten Prinzip wirkende Verbindungen, welche zur Behandlung und/oder Prophylaxe von Obesitas geeignet sind, zumindest in einem ersten orientierenden Auswahlverfahren nun ohne langwierige und teure in vivo-Tests wie Fütterungsversuche an Versuchstieren ausgewählt werden.

Diese Ergebnisse überraschen, da in früheren Untersuchungen der pharmakologischen Eigenschaften von Topiramat dessen CA-inhibitorische Wirksamkeit stets als unauffällig oder sogar als nur gering und therapeutisch unbedeutsam angegeben wurde (siehe z. B. B.E. Maryanoff et al., Journal of Medicinal Chemistry 30 (1987) 880 - 887; B. E. Maryanoff et al., Journal of Medicinal Chemistry 41 (1998) 1315 - 1343; S. J. Dodgson et al., Epilepsia 41(1)(2000) S 35 - S 39). In den vorgenannten früheren Untersuchungen wurden zur Bestimmung der CA-inhibitorischen Aktivität von Topiramat jeweils CA von verschiedenen Säugetieren enthaltende Testlösungen verwendet, welche noch verschiedene Körperbestandteile wie Blut oder Organgewebe enthielten.

In einer ersten Ausführungsform des erfindungsgemäßen Verfahrens lassen sich zur Behandlung und/oder Prophylaxe von Obesitas geeignete Verbindungen auffinden, indem solche Verbindungen als geeignet ausgewählt werden, welche die Fähigkeit besitzen, die Aktivität mindestens einer in Säugetieren vorkommenden Carboanhydrase zu hemmen. Beispielsweise kann mindestens eine zu untersuchende Verbindung mit mindestens einer Carboanhydrase kontaktiert werden und man kann anschließend diejenigen Verbindungen auf an sich bekannte Weise identifizieren, welche die Aktivität mindestens einer Carboanhydrase hemmen. Bevorzugt ist in dieser Ausführungsform die Verwendung von in Säugetieren wie Menschen oder Nagetieren, beispielsweise Ratten, Mäusen oder Meerschweinchen, vorkommenden Carboanhydrasen, insbesondere der Carboanhydrasen der Subtypen II und/oder V. Besonders bevorzugt ist die Verwendung von in Menschen vorkommenden Carboanhydrasen. Geeignete Carboanhydrasen können beispielsweise aus den vorgenannten Säugetieren isoliert und gewünschtenfalls gereinigt werden oder können vorzugsweise nach an sich bekannten chemischen oder biotechnologischen Verfahren hergestellt werden.

In einer bevorzugten Variante dieser ersten Ausführungsform kann die Änderung der Aktivität der Carboanhydrasen unter dem Einfluß der zu untersuchenden Verbindungen in einem an sich bekannten in-vitro-Enzymaktivitätstest bestimmt werden, worin die Carboanhydrasen als isolierte und von Begleitstoffen zumindest weitgehend befreite Enzyme vorliegen. Vorzugsweise werden nach chemischen oder biotechnologischen Verfahren hergestellte Carboanhydrasen verwendet, da diese in besonders reiner Form eingesetzt werden können. In-vitro-Aktivitätstests zur Bestimmung der Aktivität von Carboanhydrasen sind an sich bekannt. Zu den in-vitro-Enzymaktivitätstests, welche im Rahmen der vorliegenden Erfindung geeignet sind, Änderungen der Aktivität von Carboanhydrasen zu bestimmen, zählen beispielsweise die Messung der pH-Wert-Änderung unter dem Einfluß von Carboanhydrasen (vgl. K. M. Wilbur, N. G. Andersen, Journal of Biological Chemistry 176 (1948) 147 - 154; G. Sanyal, T. H. Maren, Journal of Biological Chemistry 256 (1981) 608 - 612), die Stop-Flow-Meßmethode (vgl. R. G. Khalifah, Journal of Biological Chemistry 246 (1971) 2561 - 2573) oder die 4-Nitrophenylacetat-Esterase-Methode (vgl. Y. Pocker, J. T. Stone, Journal of the American Chemical Society 87 (1965) 5497 - 5498). In dem letztgenannten Test wird die Geschwindigkeit der Hydrolyse von 4-Nitrophenylacetat unter dem Einfluß der zu untersuchenden Carboanhydrasen bestimmt, wobei die Eigenschaft von Carboanhydrasen, auch als Esterasen zu wirken, genutzt wird.

Erfindungsgemäß eignet sich in diesem Zusammenhang vorzugsweise ein Testverfahren zur Bestimmung der CA-inhibitorischen Eigenschaften von Verbindungen, welches beschrieben wird von C. T. Supuran et al., European Journal of Medicinal Chemistry 33 (1998) 577 - 594 (vgl. insbesondere S. 592; nachfolgend zitiert als "Supuran et al.") oder von A. Scozzafava et al., Journal of Medicinal Chemistry 42 (1999) 3690 - 3700 (vgl. insbesondere S. 3697; nachfolgend zitiert als "Scozzafava et al."). Auf die vorgenannten, von Supuran et al. und von Scozzafava et al. beschriebenen Testmethoden wird hiermit im Rahmen der Offenbarung der vorliegenden Erfindung ausdrücklich Bezug genommen. Verbindungen, welche in einem der vorgenannten in vitro-Standardaktivitätstests nach Supuran et al. oder nach Scozzafava et al. IC₅₀-Konzentrationswerte von mindestens 10 µmol/l oder darunter (= höhere Wirksamkeit) aufweisen, können als geeignete CA-inhibitorisch wirksame Verbindungen (= CA-Inhibitoren) im Sinne der vorliegenden Erfindung ausgewählt werden. Sofern die Aktivität menschlicher CA-Subtypen bestimmt werden soll, können andere als die 4-Nitrophenylacetat-Esterase-Methode besser geeignet sein. Insbesondere können Methoden geeignet sein, die es erlauben, auch verhältnismäßig schnelle Reaktionsverläufe zu verfolgen.

In einem nach der bei Scozzafava et al. (vide supra) beschriebenen 4-Nitrophenylacetat-Esterase-Methode arbeitenden Enzymaktivitätstest (ursprünglich beschrieben von Y. Pocker und J. T. Stone, Biochemistry 6 (1967) 668-678) wurde im Rahmen der vorliegenden Erfindung nachgewiesen, daß Topiramat eine ausgeprägte inhibitorische Wirkung auf nach biotechnologischen Verfahren gewonnene menschliche Carboanhydrase des Subtyps II (IC₅₀ = 5 nmol/l) aufweist, und daß diese inhibitorische Wirkung deutlich stärker ist, als die durch die als Vergleichssubstanzen gemessenen klassischen CA-Inhibitoren Acetazolamid oder Methazolamid jeweils verursachte inhibitorische Wirkung. Die menschliche Carboanhydrase des Subtyps II wurde nach der bei Scozzafava et al. angegebenen Methode erhalten.

In demselben Test wurde nachgewiesen, daß Topiramat auch eine ausgeprägte inhibitorische Wirkung auf nach biotechnologischen Verfahren gewonnene Carboanhydrase des Subtyps Va von Mäusen (= mCA Va) aufweist (IC₅₀ = 74 nmol/l). Abweichend von der bei Scozzafava et al. angegebenen Versuchsvorschrift wurde in diesem Fall das Enzym mCA Va in einer Konzentration von 120 nM eingesetzt. Die mCA Va wurde nach der von H. R. Heck et al., Journal of Biological.Chemistry 269 (1994) 24742 - 24746 angegebenen Methode auf an sich bekannte Weise erhalten. Hierzu wurde der Bakterienstamm von Escherichia coli BL 21 (DES) verwendet, welcher mit einem Plasmidvektor, der die für mCA Va kodierende Sequenz unter der Kontrolle des durch Isopropyl-β-D-thiogalactopyranosid (IPTG) induzierbaren T7-Promoters enthielt, verwendet. Die Bakterienkultur wurde bei 37 °C unter Rühren in ein Ampicillin (100 µg/ml) enthaltendes Luria-Bertani-Flüssigmedium eingeimpft und ihr Wachstum wurde bei 600 nm spektrophotometrisch verfolgt. Als die Bakterienkultur in die exponentielle Wachstumsphase eingetreten war, gab man IPTG in einer Endkonzentration von 1 mmol/l zu. Nach 3 Stunden Inkubationszeit (37 °C unter Rühren) wurde die Bakterienkultur bei 7000 x g 15 min. lang zentrifugiert und der Überstand wurde verworfen. Das resultierende Pellet wurde in 0,1 Vol doppelt destilliertem Wasser aufgenommen, und man gab Lysozym (100µg/ml) hinzu. Die Zell-Lyse erfolgte unter Ultraschallbehandlung. Hierzu gab man Aliquote von 10 ml der erhaltenen Bakteriensuspension in ein oben geöffnetes Glasgefäß und behandelte jede dieser Proben 4 mal 3 min. lang mit Ultraschall. Nach jedem Schallpuls wurde die Absorption der Proben bei 600 nm bestimmt, wofür man je 100 µl einer Probe mit 900 µl doppelt destilliertem Wasser verdünnte. Der Endpunkt war erreicht, als der Wert der 600 nm-Absorption einer Probe bei etwa 1/10 des Ausgangswertes lag. Nach erfolgter Zell-Lyse wurde CaCl₂-Bindungspuffer (Fa. Stratagene) zugegeben, und das erhaltende Zell-Lysat wurde zur Reinigung auf eine Calmodulin-Affinitäts-Harzsäule gegeben. Die Reinigung beruht auf der hohen Affinität der an das Harz gebundenen Calmodulin-Domäne zu dem Calmodulin-Binding Peptide-tag, welcher am N-terminalen Ende des exprimierten mCA Va-Proteins vorhanden ist. Die Reinigung erfolgte auf an sich bekannte Weise (vgl. Handbuch "Affinity LIC Cloning and Protein Purification Kit Manual" der Fa. Stratagene).

In einer zweiten Ausführungsform des erfindungsgemäßen Verfahrens lassen sich zur Behandlung und/oder Prophylaxe von Obesitas geeignete Verbindungen auffinden, indem man solche Verbindungen auswählt, welche die Fähigkeit besitzen, eine Verminderung der Menge von im Citronensäurezyklus isolierter lebender Säugetierzellen gebildeten Stoffwechselprodukten oder von lipidischen Folgeprodukten des Citronensäurezyklusses zu bewirken. Als Stoffwechselprodukte des Citronensäurezyklusses, deren Menge sich unter dem Einfluß der zu untersuchenden Verbindungen meßbar vermindert, eignen sich säurelösliche Stoffwechselprodukte wie Citrat, Maleat, Fumarat, und/oder α-Ketoglutarat. Citrat ist bevorzugt. Weiterhin eignen sich als Stoffwechselprodukte lipidische Folgeprodukte des Citronensäurezyklusses, wie freie Fettsäuren. Auch in dieser zweiten Ausführungstorm wird letztlich die Fähigkeit der zu untersuchenden Verbindungen bestimmt, die Aktivität mindestens einer in Säugetieren vorkommenden Carboanhydrase zu hemmen - jedoch ist das verwendete Testmodell anders angelegt als in der erstgenannten Ausführungsform. Dem Verfahren der zweiten Ausführungsform liegt das Prinzip zu Grunde, auf an sich bekannte Weise die Aufnahme von Radioaktivität aus mit dem ¹⁴C-Isotop markierten Substraten des Citronensäurezyklusses in metabolische Zwischenprodukte oder Folgeprodukte des Citronensäurezyklusses isolierter, lebender Säugetierzellen zu bestimmen und die gefundenen Ergebnisse mit den Ergebnissen zu vergleichen, welche unter ansonsten gleichen Bedingungen, aber unter dem Einfluß CA-inhibitorisch wirkender Verbindungen erzielt wurden. Vorzugsweise werden in dieser Verfahrensvariante isolierte lebende Zellen von Nagetieren wie Ratten, Mäusen oder Meerschweinchen oder von Menschen verwendet. Menschliche Zellen sind bevorzugt. Sofern Zellen von Nagetieren verwendet werden, kommen Adipozyten oder Hepatozyten in Frage. Adipozyten von Nagetieren sind bevorzugt. Sofern menschliche Zellen verwendet werden, können Adipozyten oder Hepatozyten verwendet werden. Menschliche Hepatozyten sind bevorzugt. Die in dieser Verfahrensvariante verwendeten Zellen von Säugetieren können nach üblichen Züchtungsund/oder Klonierungsverfahren erhalten werden. Es können natürliche oder biotechnologisch modifizierte Säugetierzellen verwendet werden. Sofern die Aufnahme von Radioaktivität in säurelösliche Zwischenprodukte des Citratzyklusses bestimmt werden soll, wird als ¹⁴C-markiertes Substrat vorzugsweise ¹⁴C-Hydrogencarbonat (= NaH[¹⁴C]O₃) verwendet. Sofern die Aufnahme von Radioaktivität in lipidische Folgeprodukte des Citratzyklusses bestimmt werden soll, wird als ¹⁴C-markiertes Substrat vorzugsweise [U-¹⁴C]Glucose verwendet,

Eine bevorzugte Variante dieser zweiten Ausführungsform zur Bestimmung der Fähigkeit von Verbindungen, die DNL in Säugetieren zu hemmen, wird von S. A. Hazen et al. in FASEB Journal 10(4) (1996) 481 - 490 (im folgenden zitiert als "Hazen et al.") angegeben. Auf die vorgenannte, von Hazen et al. beschriebene Testmethode wird hiermit im Rahmen der Offenbarung der vorliegenden Erfindung ausdrücklich Bezug genommen. Verbindungen, welche in einem vorgenannten Test nach Hazen et al. den Einbau von Radioaktivität aus ¹⁴C-markierten Substanzen, welche als Vorstufen im Citronensäurezyklus dienen können, beispielsweise den Einbau von ¹⁴C-Hydrogencarbonat in Citrat, Maleat, Fumarat und/oder α-Ketoglutarat, mit einem IC₅₀-Wert von mindestens 10 µmol/l oder darunter (= höhere Wirksamkeit) signifikant hemmen, können als geeignete Verbindungen im Sinne der vorliegenden Erfindung ausgewählt werden.

In einem dem bei Hazen et al. beschriebenen Testmodell entsprechenden Testmodell, welches im Rahmen der vorliegenden Erfindung durchgeführt wurde, zeigte Topiramat eine ausgeprägte hemmende Wirkung auf die Entstehung säurelöslicher Stoffwechselprodukte des Citronensäurezyklusses von nach biotechnologischen Verfahren gewonnenen Ratten-Adipozyten der Zellinie 3T3-F442A. Diese hemmende Wirkung von Topiramat (IC₅₀ = 348 nmol/l) war deutlich ausgeprägter als die Wirkung des als Vergleichssubstanz gemessenen klassischen CA-Inhibitors Ethoxzolamid.

In einer besonders bevorzugten Variante des erfindungsgemäßen Verfahrens zum Auffinden von Verbindungen, welche zur Behandlung und/oder Prophylaxe von Obesitas geeignet sind, werden solche Verbindungen ausgewählt, welche in der vorgenannten ersten Ausführungsform des Verfahrens, insbesondere in einem vorgenannten in-vitro-Enzymaktivitätstest, als geeignet zur Hemmung mindestens einer in Säugetieren vorkommenden Carboanhydrase ausgewählt wurden und welche zusätzlich auch in der vorgenannten zweiten Ausführungsform des Verfahrens als geeignet ausgewählt wurden, eine Verminderung der Menge von im Citronensäurezyklus isolierter lebender Säugetierzellen gebildeten Stoffwechselprodukten zu bewirken. In der ersten Ausführungsform des Verfahrens kann rasch und effektiv die Fähigkeit von Verbindungen überprüft werden, in Säugetieren vorkommende Carboanhydrasen zu hemmen. Die zweite Ausführungsform des Verfahrens liefert u. a. Anhaltspunkte dafür, ob die untersuchten Verbindungen auch die Fähigkeit besitzen, in Mitochondrien lebender Säugetierzellen einzudringen, wo CA V lokalisiert ist. Zur Auswahl geeigneter Verbindungen können die erste und die zweite vorgenannte Ausführungsform parallel oder in beliebiger Reihenfolge nacheinander durchgeführt werden.

Erfindungsgemäß eignen sich Verbindungen, welche die Fähigkeit besitzen, die DNL in Säugetieren zu hemmen, zur Herstellung von Arzneimitteln für die Behandlung und/oder Prophylaxe von Obesitas. Dabei werden solche Verbindungen ausgewählt, welche die Fähigkeit besitzen, mindestens eine in Säugetieren vorkommende Carboanhydrase zu hemmen. Die ausgewählten Verbindungen müssen selbstverständlich physiologisch verträglich sein und die allgemein an Arzneimittelwirkstoffe gestellten Forderungen, betreffend z. B. Sicherheit und Verträglichkeit, erfüllen. Bevorzugt ist die Verwendung von Verbindungen, welche in Säugetieren vorkommende Carboanhydrasen der Subtypen II und/oder V zu hemmen vermögen. Besonders bevorzugt sind Verbindungen, welche spezifisch CA II und/oder CA V, insbesondere CA V, zu hemmen vermögen.

Vorbekannte Verbindungen mit CA-inhibitorischer Wirkung, welche bei längerer Verabreichung eine Reduzierung des Körpergewichtes der Patienten zur Folge haben, beispielsweise Topiramat, weisen auch ausgeprägte, auf das ZNS gerichtete Wirkungen wie antikonvulsive Wirkungen auf. Für Verbindungen, welche zur Verabreichung über längere Zeiträume mit dem Ziel der Behandlung und/oder Prophylaxe von Obesitas vorgesehen sind, sind auf das ZNS gerichtete Begleitwirkungen oft unerwünscht. Verbindungen, welche nach dem vorgenannten erfindungsgemäßen Verfahren als geeignet zur Behandlung und/oder Prophylaxe von Obesitas ausgewählt wurden, können daher zusätzlich auf Wirkungen hin untersucht werden, welche auf das ZNS gerichtet sind. Beispielsweise können die Verbindungen auf gegebenenfalls vorhandene antikonvulsive Eigenschaften untersucht werden. Zur Untersuchung von Verbindungen auf antikonvulsive Eigenschaften eignet sich beispielsweise der sogenannte "Supramaximal-Elektroschocktest" (= SES-Test, gelegentlich auch als "Maximal-Elektroschocktest", MES-Test bezeichnet) gemäß G. Chen et al., Proceedings of the Society for Experimental Biology and Medicine, 87 (1954) 334 - 339 (im folgenden als "Chen et al." zitiert) und J. E. P. Toman et al., Journal of Neurophysiology 9 (1946) 231 (im folgenden als "Toman et al." zitiert). Auf die vorgenannte, von Chen et al. und von Toman et al. beschriebene Testmethode wird hiermit im Rahmen der Offenbarung der vorliegenden Erfindung ausdrücklich Bezug genommen. So sollten Verbindungen, welche über längere Zeiträume mit dem Ziel der Behandlung und/oder Prophylaxe von Obesitas an Patienten verabreicht werden sollen, in dem vorgenannten SES-Test nach Chen et al. und nach Toman et al. im wesentlichen unwirksam sein und sollten vorzugsweise auch in höheren Dosierungen von zumindest 100 mg/kg p. o. keine nach den für diesen Test üblichen Kriterien als signifikant anzusehende Wirkungen aufweisen (i. e. "Protective Dose" PD₅₀ nach G. Chen et al. ≥100 mg/kg p. o.; die von G. Chen et al. angegebenen PD₅₀-Werte entsprechen dabei im wesentlichen der gebräuchlicheren Dosisangabe als "Minimal Effektive Dosis", MED). Verbindungen, welche in dem vorgenannten Verfahren zum Auffinden von Verbindungen als geeignet ausgewählt wurden und welche zusätzlich in dem vorgenannten MES-Test nach Chen et al. im wesentlichen unwirksam sind, sind besonders geeignet zur Herstellung von Arzneimitteln für die Behandlung und/oder Prophylaxe von Obesitas. Vorgenannte SES-(bzw. MES-)Tests sind pharmakologische Standardtests und können bei entsprechenden Serviceanbietern (z. B. bei Firma "Panlabs") als Routinemethoden durchgeführt werden.

Die nach dem erfindungsgemäßen Verfahren als zur Behandlung und/oder Prophylaxe von Obesitas geeignet aufgefundenen Verbindungen können üblicherweise als Arzneimittel mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester oder flüssiger Trägerstoffe, wie z. B. Milchzucker, Stärke oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln. Erfindungsgemäß geeignete pharmazeutische Zubereitungen sind beispielsweise auch aus der EP 0 138 441 A2 sowie aus der WO 98/00130 bekannt.

## Patentansprüche

1. Verfahren zum Auffinden von Verbindungen, welche zur Behandlung und/oder Prophylaxe von Obesitas geeignet sind, **dadurch gekennzeichnet, daß** man solche Verbindungen auswählt, welche die Fähigkeit besitzen, die Aktivität mindestens einer in Säugetieren vorkommenden Carboanhydrase zu hemmen.

2. Verfahren nach Anspruch 1, worin man mindestens eine Verbindung mit mindestens einer Carboanhydrase kontaktiert und anschließend diejenigen Verbindungen identifiziert, welche die Aktivität mindestens einer Carboanhydrase hemmen.

3. Verfahren nach Anspruch 1, worin in Nagetieren oder Menschen vorkommende Carboanhydrasen verwendet werden.

4. Verfahren nach Anspruch 3, worin in Menschen vorkommende Carboanhydrasen verwendet werden.

5. Verfahren nach Anspruch 1, worin in Säugetieren, insbesondere Menschen, vorkommende Carboanhydrasen der Subtypen II und/oder V verwendet werden.

6. Verfahren nach Anspruch 5, worin in Säugetieren, insbesondere Menschen, vorkommende Carboanhydrasen des Subtyps V verwendet werden.

7. Verfahren nach Anspruch 2, worin die Carboanhydrasen als nach chemischen oder biotechnologischen Verfahren gewonnene isolierte Enzyme vorliegen und worin die Änderung der Aktivität der Carboanhydrasen unter dem Einfluß der Verbindungen in einem in-vitro-Enzymaktivitätstest bestimmt wird.

8. Verfahren nach Anspruch 1, worin solche Verbindungen als geeignet ausgewählt werden, welche die Fähigkeit besitzen, eine Verminderung der Menge von im Citronensäurezyklus isolierter, lebender Säugetierzellen gebildeten Stoffwechselprodukten zu bewirken.

9. Verfahren nach Anspruch 8, worin die Verminderung der Menge von im Citronensäurezyklus der Zellen gebildetem Citrat, Maleat, Fumarat, α-Ketoglutarat und/oder die Verminderung der Bildung lipidischer Folgeprodukte des Citronensäurezyklusses unter dem Einfluß der Verbindungen bestimmt wird.

10. Verfahren nach Anspruch 8, worin isolierte lebende Zellen von Nagetieren oder Menschen verwendet werden.

11. Verfahren nach Anspruch 10, worin Adipozyten oder Hepatozyten verwendet werden.

12. Verfahren zum Auffinden von Verbindungen, welche zur Behandlung und/oder Prophylaxe von Obesitas geeignet sind, **dadurch gekennzeichnet, daß** man Verbindungen auswählt, welche in einem Verfahren nach Anspruch 7 und zusätzlich in einem Verfahren nach Anspruch 8 jeweils als geeignete Verbindungen identifiziert wurden.

13. Verfahren zum Auffinden von Verbindungen, welche zur Behandlung und/oder Prophylaxe von Obesitas geeignet sind, worin man Verbindungen auswählt, welche in einem Verfahren nach Anspruch 1 als geeignete Verbindungen identifiziert wurden und welche zusätzlich in einem Standardtest zur Bestimmung von antikonvulsiven Eigenschaften als unwirksam identifiziert wurden.

14. Verwendung von Verbindungen, welche die Fähigkeit besitzen, mindestens eine in Säugetieren vorkommende Cärboanhydrase zu hemmen, zur Herstellung eines Arzneimittels für die Behandlung und/oder Prophylaxe von Obesitas.

15. Verwendung nach Anspruch 14 von Verbindungen, welche die Fähigkeit besitzen, in Säugetieren vorkommende Carboanhydrasen der Subtypen II und/oder V zu hemmen.

16. Verwendung nach Anspruch 14 von Verbindungen, welche frei sind von antikonvulsiven Eigenschaften.

17. Verwendung von Verbindungen, welche erstmals nach einem Verfahren gemäß Anspruch 1 als zur Behandlung und/oder Prophylaxe von Obesitas geeignet identifiziert wurden, zur Herstellung eines Arzneimittels für die Behandlung und/oder Prophylaxe von Obesitas.

## Claims

1. A method of discovering compounds suitable for the treatment and/or prophylaxis of obesity, **characterised in that** those compounds are selected which are capable of inhibiting the activity of at least one carboanhydrase occurring in mammals.

2. A method according to claim 1, wherein at least one compound is brought into contact with at least one carboanhydrase, and those compounds which inhibit the activity of at least one carboanhydrase are then identified.

3. A method according to claim 1, wherein carboanhydrases occurring in rodents or humans are used.

4. A method according to claim 3, wherein carboanhydrases occurring in humans are used.

5. A method according to claim 1, wherein carboanhydrases of subtypes II and/or V occurring in mammals, especially humans, are used.

6. A method according to claim 5, wherein carboanhydrases of subtype V occurring in mammals, especially humans, are used.

7. A method according to claim 2, wherein the carboanhydrases are present as isolated enzymes obtained by chemical or biotechnological methods, and wherein the change in activity of the carboanhydrases under the influence of the compounds is determined in an in vitro enzyme activity test.

8. A method according to claim 1, wherein the compounds selected as suitable are those which are capable of reducing the amount of metabolic products formed in the citric acid cycle of isolated living mammalian cells.

9. A method according to claim 8, wherein the reduction in the amount of citrate, maleate, fumarate and α-ketoglutarate formed in the citric acid cycle of the cells and/or the reduction of the formation of lipidic subsequent products of the citric acid cycle under the influence of the compounds is determined.

10. A method according to claim 8, wherein isolated living cells of rodents or humans are used.

11. A method according to claim 10, wherein adipocytes or hepatocytes are used.

12. A method of discovering compounds suitable for the treatment and/or prophylaxis of obesity, **characterised in that** compounds are selected which have been identified as suitable compounds in a method according to claim 7 and additionally in a method according to claim 8.

13. A method of discovering compounds suitable for the treatment and/or prophylaxis of obesity, wherein compounds are selected which have been identified as suitable compounds in a method according to claim 1, and which have additionally been identified as ineffective in a standard test for the determination of anticonvulsant properties.

14. The use of compounds which are capable of inhibiting at least one carboanhydrase occurring in mammals, for the preparation of a drug for the treatment and/or prophylaxis of obesity.

15. The use according to claim 14 of compounds which are capable of inhibiting carboanhydrases of subtypes II and/or V occurring in mammals.

16. The use according to claim 14 of compounds which are free of anticonvulsant properties.

17. The use of compounds which have been identified for the first time by a method according to claim 1 as suitable for the treatment and/or prophylaxis of obesity for the preparation of a drug for the treatment and/or prophylaxis of obesity.

## Revendications

1. Procédé de détection de composés appropriés pour le traitement et/ou la prophylaxie de l'obésité, **caractérisé en ce que** l'on sélectionne des composés qui possèdent l'aptitude à inhiber l'activité d'au moins une carboanhydrase présente chez les mammifères.

2. Procédé selon la revendication 1, dans lequel on met en contact au moins un composé avec au moins une carboanhydrase et on identifie ensuite les composés qui inhibent l'activité d'au moins une carboanhydrase.

3. Procédé selon la revendication 1, dans lequel on utilise des carboanhydrases existant chez les rongeurs ou les humains.

4. Procédé selon la revendication 3, dans lequel on utilise des carboanhydrases présentes chez les humains.

5. Procédé selon la revendication 1, dans lequel on utilise des carboanhydrases des sous-types II et/ou V présentes chez les mammifères, en particulier chez les humains.

6. Procédé selon la revendication 5, dans lequel on utilise des carboanhydrases du sous-type V présentes chez les mammifères, en particulier chez les humains.

7. Procédé selon la revendication 2, dans lequel les carboanhydrases se présentent en tant qu'enzymes isolées obtenues par des voies chimiques ou biotechnologiques et dans lequel on détermine la modification de l'activité des carboanhydrases sous l'influence des composés lors d'un test d'activité enzymatique in vitro.

8. Procédé selon la revendication 1, dans lequel on sélectionne comme appropriés des composés qui possèdent l'aptitude à entraîner une réduction de la quantité de produits du métabolisme formés par des cellules de mammifères vivantes, isolées au cours du cycle de l'acide citrique.

9. Procédé selon la revendication 8, dans lequel on détermine la réduction de la quantité de citrate, de maléate, de fumarate, d'α-cétoglutarate formé par les cellules au cours du cycle de l'acide citrique et/ou la réduction de la formation de produits résultants lipidiques du cycle de l'acide citrique sous l'influence des composés.

10. Procédé selon la revendication 8, dans lequel on utilise des cellules vivantes isolées de rongeurs ou d'humains.

11. Procédé selon la revendication 10, dans lequel on utilise des adipocytes ou des hépatocytes.

12. Procédé de détection de composés appropriés pour le traitement et/ou la prophylaxie de l'obésité, **caractérisé en ce que** l'on sélectionne des composés qui ont été respectivement identifiés comme étant des composés appropriés dans un procédé selon la revendication 7 et en outre dans un procédé selon la revendication 8.

13. Procédé de détection de composés appropriés pour le traitement et/ou la prophylaxie de l'obésité, dans lequel on sélectionne des composés qui ont été identifiés comme composés appropriés dans un procédé selon la revendication 1 et qui ont en outre été identifiés dans un test standard de détermination des propriétés anti-convulsives comme étant essentiellement non efficaces.

14. Utilisation de composés qui possèdent l'aptitude à inhiber au moins une carboanhydrase présente chez les mammifères, pour fabriquer un médicament destiné au traitement et/ou à la prophylaxie de l'obésité.

15. Utilisation selon la revendication 14 de composés qui possèdent l'aptitude à inhiber les carboanhydrases des sous-types II et/ou V présentes chez les mammifères.

16. Utilisation selon la revendication 14 de composés qui sont exempts de propriétés anti-convulsives.

17. Utilisation de composés qui ont été identifiés pour la première fois par un procédé selon la revendication 1 comme appropriés pour le traitement et/ou la prophylaxie de l'obésité, pour fabriquer un médicament destiné au traitement et/ou à la prophylaxie de l'obésité.
